# EUROPEAN PATENT SPECIFICATION

(11) **EP 1 974 721 B1**
(45) Date of publication and mention of the grant of the patent: **13.07.2011**
(21) Application number: 07122822.5
(22) Date of filing: 11.12.2007
(51) Int. Cl.: A61K 9/14, A61K 47/12, A61K 9/08, A61K 31/431

(54) **Injectable sterile pharmaceutical composition with piperacillin sodium and tazobactam sodium as active principles**
Injizierbare sterile pharmazeutische Zusammensetzung mit Piperacillin-Natrium und Tazobactam-Natrium als Wirkstoffe
Composition pharmaceutique stérile injectable avec sodium de pipéracilline et sodium de tazobactan en tant que principes actifs

(30) Priority: 22.03.2007 IT MI20070568
(43) Date of publication of application: 01.10.2008
(73) Proprietor: ACS DOBFAR S.p.A., 20067 Tribiano (Milano) (IT)
(72) Inventor: CATTANEO, Angelo Giovanni, 24030, MONTE MARENZO (LC) (IT); MARSILI, Leonardo, 25122, BRESCIA (IT)
(74) Representative: Frignoli, Luigi

(56) References cited:
- EP-A- 1 759 697
- WO-A-2004/098643
- WO-A-2006/044600
- US-A1- 2005 171 077

## Description

The present invention relates to a time-stable sterile pharmaceutical composition comprising piperacillin sodium and tazobactam sodium mixed with sodium bicarbonate, this composition being soluble in water to give an injectable sterile solution highly stable with time.

EP 1759697A in the name of the present applicants describes the preparation of a stable, sterile homogeneous mixture of amorphous solid particles of piperacillin sodium and tazobactam sodium particles of substantially the same density, which is soluble in water to give a reconstituted solution with a pH of about 5.6

Such mixture is obtained by a process according to which piperacilliln and tazobactam are dissolved in acid form, at a temperature between -10°C and +25°C, in a solvent consisting of at least two chosen from the group consisting of water, acetone, methanol, isopropylalcohol, ethanol, salifying the mixture by adding suitable sodium salts chosen from the group consisting of sodium carbonate, sodium 2-ethyl-hexanoate, sodium acetate, sodium citrate, sodium lactate, sodium methylate, sodium ethylate, sterilely filtering the solution obtained, precipitating the salified mixture by feeding the sterile solution dropwise into at least one organic solvent chosen from the group consisting of isopropyl alcohol, ethyl alcohol, methyl acetate, ethyl acetate, acetone, methylene chloride at a temperature between 0°C and 50°C, filtering the saline mixture obtained and finally drying it under vacuum at a temperature between 20°C and 75°C.

During tests carried out after filing of the above mentioned application it was however observed that the said sterile solution can give rise to a precipitate or to turbidity some minutes after its preparation. This quantitatively very limited precipitate, or possible turbidity, renders the solution unsuitable for clinical use, also in consideration of the fact that administration of the drug by injection does not always immediately follow the preparation of the injectable solution, but several minutes can pass between the solution preparation and its administration to the patient.

The object of the present application is to provide an injectable solution comprising a mixture of amorphous solid particles of piperacillin sodium and tazobactam sodium which remains stable after dissolution of said solid mixture prepared according to the cited European application (dried under vacuum at 20-75°C).

This object has been achieved by adding a certain amount of sterile sodium bicarbonate i.e. between 1.1% and 1.2% on the total weight of said active agents.

In this respect, the sodium bicarbonate presents no problem during its mixing with the active principles, in contrast to other buffer agents which could be considered and which can contain water of crystallization (e.g. sodium citrate) which is potentially damaging to the stability of the dry product.

More specifically, the present invention relates to a stable injectable sterile pharmaceutical composition comprising a mixture of active components consisting of amorphous solid particles of piperacillin sodium and tazobactam sodium in a weight ratio of about 8:1, characterised by also comprising sterile sodium bicarbonate in a quantity between 1.1% and 1.2% on the total weight of said active components, the pharmaceutical composition being soluble in water to give reconstituted solutions of pH between 6.0 and 6.5 having high stability with time.

The preparation process for the aforesaid composition is characterised by adding between 1.1 wt% and 1.2 wt% of sterile sodium bicarbonate to, and carefully mixing it with, a mixture of active components consisting of amorphous solid particles of piperacillin sodium and tazobactam sodium in a weight ratio of about 8:1 obtained by dissolving at least one active principle in acid form, at a temperature between -10°C and +25°C, in a solvent consisting of at least two chosen from the group consisting of water, acetone, methanol, isopropylalcohol, ethanol, salifying the mixture by adding suitable sodium salts chosen from the group consisting of sodium carbonate, sodium 2-ethyl-hexanoate, sodium acetate, sodium citrate, sodium lactate, sodium methylate, sodium ethylate, sterilely filtering the solution obtained, precipitating the salified mixture by feeding the sterile solution dropwise into at least one organic solvent chosen from the group consisting of isopropyl alcohol, ethyl alcohol, methyl acetate, ethyl acetate, acetone, methylene chloride at a temperature between 0°C and 50°C, filtering the saline mixture obtained and finally drying it under vacuum at a temperature between 20°C and 75°C.

The characteristics of the composition of the invention and of its preparation process will be more apparent from the ensuing description of two non-limiting examples thereof.

### Example 1

10.35 g sodium 2-ethylhexanoate are dissolved in a mixture of 25.5 ml water and 45 ml methanol cooled to 0°C. 30 g piperacillin monohydrate are then added and the mixture maintained under stirring until complete dissolution. 2.25 g sodium 2-ethylhexanoate are added followed by 3.75 g tazobactam acid. The mixture is maintained under stirring for 90 min. between 0°C and +5°C, the suspension obtained is filtered and, while maintaining at the same temperature, is fed dropwise over at least 30 min into 600 ml isopropyl alcohol maintained at 35°C. Agitation is continued for 30 min at 35°C, the mixture filtered, the product washed with isopropyl alcohol and dried under vacuum at 75°C for 24 h : 27,9 g are obtained having
K.F.: 3.5%.
Isopropyl alcohol: substantially absent
pH of reconstituted aqueous solution: 5.6.
The methanol used in the mixture for dissolving the sodium 2-ethylhexanoate may be partially or almost totally replaced by isopropyl alcohol.
In sterile environment, 0.32 g sterile sodium bicarbonate are added to the abovementioned solid sterile mixture (27,9 g) containing piperacillin and tazobactam as sodium salts, and carefully mixed in order to obtain an homogeneous solid sterile mixture.
1.264 g of the above obtained solid mixture are dissolved in 2 ml water: pH of reconstituted aqueous solution is 6.1 while no degradation of piperacillin is observed.

### Example 2

10.87 g sodium 2-ethylhexanoate are dissolved in a mixture of 31.5 ml water and 31.5 ml methanol cooled to 0°C. 31.6 g acid piperacillin monohydrate are then added and the mixture maintained under stirring until complete dissolution. 3.95 g tazobactamacid are added followed by 2.36 g sodium 2-ethylhexanoate. The walls of the reaction flask are then washed with 16 ml methanol at 0°C. The mixture is maintained under agitation for 90 min between 0°C and +5°C, the solution obtained is filtered and, while maintaining at the same temperature, is fed dropwise over 45 min into 630 ml isopropyl alcohol maintained at 35°C. Stirring is continued for 30 min at 35°C, the mixture cooled to +15°C and stirred for 30 min. It is filtered, the product washed with 31.5 ml isopropyl alcohol and dried under vacuum at 70°C for 90 min. The product (29.3 g) is a solid, sterile mixture of piperacillin sodium salt and tazobactam sodium salt. 0.34 g sterile sodium bicarbonate powder are added and carefully mixed in sterile environment with the above mentioned product. 1.265 g of the so obtained sterile, solid mixture are dissolved in 2 ml water: pH of the reconstituted solution is 6.4 while no degradation of piperacillin is observed.

## Claims

1. A stable injectable sterile pharmaceutical composition comprising a mixture of active components consisting of amorphous solid particles of piperacillin sodium and tazobactam sodium in a weight ratio of about 8:1, **characterised by** also comprising sterile sodium bicarbonate in a quantity between 1.1 % and 1.2% on the total weight of said active components, the pharmaceutical composition being soluble in water to give reconstituted solutions of pH between 6.0 and 6.5 having high stability with time.

2. A preparation process for an injectable sterile pharmaceutical composition as claimed in claim 1, **characterised by** adding between 1.1 wt% and 1.2 wt% of sterile sodium bicarbonate to, and carefully mixing it with, a mixture of active components consisting of amorphous solid particles of piperacillin sodium and tazobactam sodium in a weight ratio of about 8:1 obtained by dissolving at least one active principle in acid form, at a temperature between -10°C and +25°C, in a solvent consisting of at least two chosen from the group consisting of water, acetone, methanol, isopropylalcohol, ethanol, salifying the mixture by adding suitable sodium salts chosen from the group consisting of sodium carbonate, sodium 2-ethyl-hexanoate, sodium acetate, sodium citrate, sodium lactate, sodium methylate, sodium ethylate, sterilely filtering the solution obtained, precipitating the salified mixture by feeding the sterile solution dropwise into at least one organic solvent chosen from the group consisting of isopropyl alcohol, ethyl alcohol, methyl acetate, ethyl acetate, acetone, methylene chloride at a temperature between 0°C and 50°C, ^{.}filtering the saline mixture obtained and finally drying it under vacuum at a temperature between 20°C and 75°C.

## Patentansprüche

1. Stabile injizierbare sterile pharmazeutische Zusammensetzung, umfassend ein Gemisch von aktiven Komponenten, bestehend aus amorphen Feststoffpartikeln aus Piperacillin-Natrium und Tazobactam-Natrium in einem Gewichtsverhältnis von etwa 8:1, **dadurch gekennzeichnet, dass** sie auch steriles Natriumbicarbonat in einer Menge von zwischen 1,1% und 1,2%, bezogen auf das Gesamtgewicht der aktiven Komponenten, umfasst, wobei die pharmazeutische Zusammensetzung in Wasser löslich ist, so dass rekonstituierte Lösungen mit einem pH zwischen 6,0 und 6,5 erhalten werden, die eine hohe Stabilität über die Zeit haben.

2. Verfahren zur Herstellung einer injizierbaren sterilen pharmazeutischen Zusammensetzung, wie sie in Anspruch 1 beansprucht ist, **gekennzeichnet durch** Zusetzen von zwischen 1,1 Gew.-% und 1,2 Gew.-% sterilem Natriumbicarbonat zu und vorsichtiges Vermischen mit einem Gemisch von aktiven Komponenten, bestehend aus amorphen Feststoffpartikeln aus Piperacillin-Natrium und Tazobactam-Natrium in einem Gewichtsverhältnis von etwa 8:1, das erhalten wurde, indem wenigstens ein Wirkstoff in Säureform bei einer Temperatur zwischen -10°C und +25°C in einem Lösungsmittel, bestehend aus wenigstens zwei, ausgewählt aus der Gruppe, bestehend aus Wasser, Aceton, Methanol, Isopropylalkohol, Ethanol, gelöst wird, Überführen des Gemisches in Salz **durch** Zugabe von geeigneten Natriumsalzen, ausgewählt aus der Gruppe, bestehend aus Natriumcarbonat, Natrium-2-ethylhexanoat, Natriumacetat, Natriumcitrat, Natriumlactat, Natriummethylat, Natriumethylat; Sterilfiltrieren der erhaltenen Lösung; Präzipitieren des als Salz vorliegenden Gemisches **durch** Einführen der sterilen Lösung tropfenweise in wenigstens ein organisches Lösungsmittel, ausgewählt aus der Gruppe, bestehend aus Isopropylalkohol, Ethylalkohol, Methylacetat, Ethylacetat, Aceton, Methylenchlorid, bei einer Temperatur zwischen 0°C und 50°C, Filtrieren des erhaltenen Salzgemisches und schließlich Trocknung desselben unter Vakuum bei einer Temperatur zwischen 20°C und 75°C.

## Revendications

1. Composition pharmaceutique stable, stérile et injectable, comprenant un mélange de composants actifs constitués de particules solides amorphes de pipéracilline sodique et de tazobactam sodique à un rapport pondéral d'environ 8 : 1, **caractérisée en ce qu'**elle comprend également du bicarbonate de sodium stérile en une quantité comprise entre 1,1 % et 1,2 % sur la base du poids total desdits composants actifs, la composition pharmaceutique étant soluble dans l'eau pour donner des solutions reconstituées ayant un pH compris entre 6,0 et 6,5 ayant une grande stabilité dans le temps.

2. Procédé de préparation pour une composition pharmaceutique stérile injectable selon la revendication 1, **caractérisé par** l'ajout de 1,1 % en poids à 1,2 % en poids de bicarbonate de sodium stérile à, et son mélange intime avec, un mélange de composants actifs constitués de particules solides amorphes de pipéracilline sodique et de tazobactam sodique à un rapport pondéral d'environ 8 : 1, obtenues par la dissolution d'au moins un principe actif sous forme acide, à une température comprise entre -10 °C et +25 °C, dans un solvant constitué d'au moins deux solvants choisis dans le groupe constitué par l'eau, l' acétone, le méthanol, l' alcool isopropylique, l'éthanol, la salification du mélange par l'ajout de sels de sodium appropriés choisis dans le groupe constitué par le carbonate de sodium, le 2-éthyl-hexanoate de sodium, l' acétate de sodium, le citrate de sodium, le lactate de sodium, le méthylate de sodium, l'éthylate de sodium, la filtration stérile de la solution obtenue, la précipitation du mélange salifié par l'ajout goutte à goutte de la solution stérile à au moins un solvant organique choisi dans le groupe constitué par l' alcool isopropylique, l' alcool éthylique, l' acétate de méthyle, l' acétate d'éthyle, l'acétone, le chlorure de méthylène, à une température comprise entre 0 °C et 50 °C, la filtration du mélange salé obtenu et finalement son séchage sous vide à une température comprise entre 20 °C et 75 °C.
